# EUROPEAN PATENT APPLICATION

(11) **EP 4 763 145 A2**
(43) Date of publication of application: **24.06.2026**
(21) Application number: 26168734.7
(22) Date of filing: 03.07.2024
(51) Int. Cl.: A61F 2/32

(54) **FINISHING APPARATUS FOR BIOMEDICAL PROSTHESIS COMPONENTS, IN PARTICULAR FOR HIP PROSTHESIS RODS**

(30) Priority: 06.07.2023 IT 202300014169
(62) Divisional of application: 24186369.5
(71) Applicant: Robot Service S.r.l., 25013 Carpenedolo, Brescia (IT)
(72) Inventor: ZANINELLI, Devis, 25013 Carpenedolo, Brescia (IT)
(74) Representative: Pulieri, Gianluca Antonio

(57) **Abstract**

A finishing apparatus (100) with a double robotic station performs grinding and polishing processes on a hip prosthesis rod, made of steel, ferrous alloy, titanium or titanium alloy. The finishing apparatus (100) comprises a loading unit (202), a grinding station (200), a shifter unit (400), and a polishing station (300). [Fig. 3a]

## Description

### Technical Field

The present invention belongs to the field of machines for finishing hip prostheses, i.e., automated machines intended to perform processes such as grinding and polishing of semi-finished components for hip prostheses, generally made of steel, ferrous alloys or titanium alloys, obtained for example by forging.

### Prior art

The biomedical prostheses, now widely used, are known to have a very high production cost; this is essentially due to the materials used and the production processes required to obtain the desired performance. Very often, the semi-finished products are made of steel, ferrous alloys or titanium alloys, by forging, and then subjected to surface finishing processes, which are essential to obtain the tolerances and surface condition for biomedical use. Since the origins of modern implantology, and very often still now, such finishing processes are carried out manually, because of the automation difficulties due to the complex shape of the semi-finished products, the surface finish degree to be achieved, and the dimensional and geometric tolerances prescribed.

In order to keep production costs down and to meet the increasing demand as well, manufacturers of automated finishing apparatuses have provided some apparatuses suitable for processing components for biomedical prostheses. Such apparatuses are generally designed for specific components, for example parts of hip prostheses or parts of knee prostheses, due to the peculiar shape of each of these and the desired finishing processes.

However, the need is still felt to have an apparatus for carrying out finishing processes on hip prosthesis components that fully meets the needs of the industry, and in particular the needs to reduce production times, obtain the desired surface finish degree, and comply with the dimensional and geometric tolerances prescribed.

### Object of the invention

It is the object of the present invention to provide a finishing apparatus for biomedical prosthesis components, and in particular for hip prosthesis rods, which allows overcoming the drawbacks mentioned with reference to the prior art and meeting the aforesaid needs.

Such an object is achieved by a loading unit, a shifter unit, and a finishing apparatus according to the following claims.

### Brief description of the drawings

The features and advantages of the loading and unloading unit according to the present invention will become apparent from the following description, given by way of non-limiting example, according to the accompanying drawings, in which:
- Figure 1 depicts a rod of a hip prosthesis;
- Figure 2 depicts a cap for a neck of the rod in Figure 1;
- Figures 3a to 3g show the work steps of a finishing apparatus according to an embodiment of the present invention;
- Figures 4a to 4c show a loading unit of the finishing apparatus according to the present invention;
- Figures 5a to 5d depict an example of positioning means of the loading unit and the steps of manually loading a loading carriage of the loading unit;
- Figures 6a and 6b depict the loading carriage of the loading unit, provided with semi-processed rods;
- Figures 7a to 7d show steps of picking from the loading carriage;
- Figure 8 shows a first checking station of the finishing apparatus;
- Figure 9 depicts a shifter unit of the finishing apparatus;
- Figures 10a to 10e depict second positioning means of the shifter unit and the steps of depositing on a shifter carriage of the shifter unit;
- Figures 11a to 11d depict said second positioning means of the shifter unit and the steps of picking from the shifter carriage of the shifter unit;
- Figures 12a to 12c show a second checking station and related work steps;
- Figure 13 shows a waste store of the finishing apparatus;
- Figures 14a and 14b depict a third checking station of the finishing apparatus;
- Figure 15 depicts a finished piece store of the finishing apparatus;
- Figure 16 depicts an alternative embodiment of a loading carriage.

### Detailed description of the invention

### Semi-finished rod

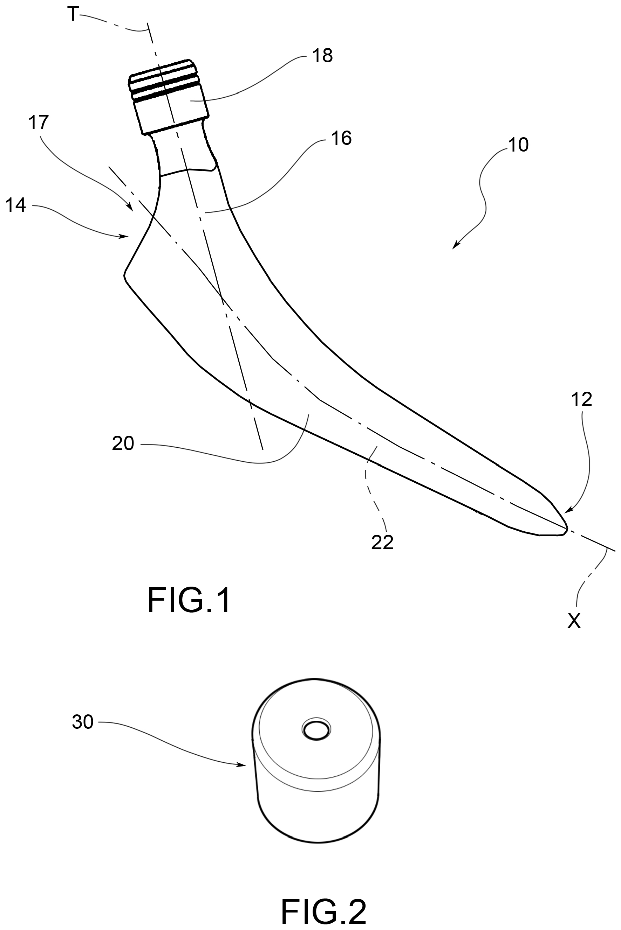

For clarity of explanation, reference will be made below to a semi-finished rod for hip prostheses, of the type depicted in Figure 1. The invention is however applicable to any component of a biomedical prosthesis, unless specified otherwise in the claims.

Generally, a hip prosthesis comprises a rod, for example made of steel, ferrous alloy, titanium or titanium alloy, intended to be implanted in the femur, a hemispherical small head, for example made of ceramic or polyethylene material, applied to the head of the rod, a cotyloid cavity (consisting of a cup generally made of titanium and a body made of ceramic material or polyethylene having a hemispherical seat), intended to be implanted in the hip; the small head is accommodated in the seat of the cotyloid cavity.

The semi-processed rod 10 is preferably made by forging, in a single body; the semi-processed rod 10 is subjected to finishing processes to obtain the finished rod, such as in the case of a rod made of steel or ferrous alloy, for example, or subjected to further processes, such as sintering to create a surface layer which promotes osseointegration, for example, as in the case of a rod made of titanium or titanium alloy.

The semi-processed rod 10 has a twisted shape mainly extending along an arched direction X, having a neck 16 protruding from the arched direction X between a preferably pointed lower end 12 and an upper portion 14, becoming thinner compared to the rest of the body, and a shoulder 17 due to the neck thinning. The neck 16 finally comprises a head 18, usually cylindrical with a central axis T, to be coupled to the hemispherical small head. Generally, the semi-processed rod 10 has a first face 20 and a second face 22, which determine the usually constant thickness thereof.

For the protection of the head 18 during the finishing processes, a cap 30 is used, preferably made of plastic material, e.g., PolyEther Ether Ketone (PEEK), adapted to be fitted with interference, e.g., snap-fitted, onto the head 18.

### Finishing apparatus

A finishing apparatus 100 according to the present invention is suitable for carrying out finishing processes on the semi-processed rod 10.

The finishing apparatus 100 comprises a grinding station 200 and a polishing station 300 which are separate, placed side by side and cooperating; the semi-processed rod is first subjected to grinding processes in the grinding station 200 and then to polishing processes in the polishing station 300.

The grinding station 200 preferably comprises a grinding booth 201 which delimits it peripherally and preferably at the top.

The finishing apparatus 100 comprises a loading unit 202 for introducing a plurality of semi-processed rods 10 into the grinding station 200; for this purpose, the loading unit 202 comprises a loading carriage 204 being movable, e.g., translatable, for example by means of pneumatic actuation.

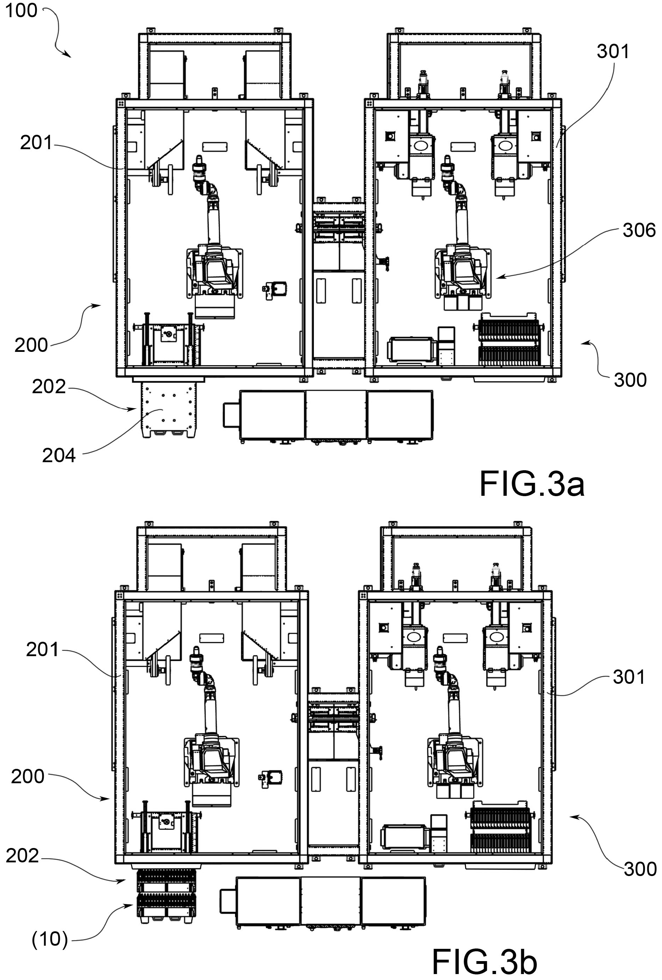

In an initial step of a work process of the finishing apparatus 100, the loading carriage 204 is placed outside the grinding booth 201 and is empty (Figure 3a).

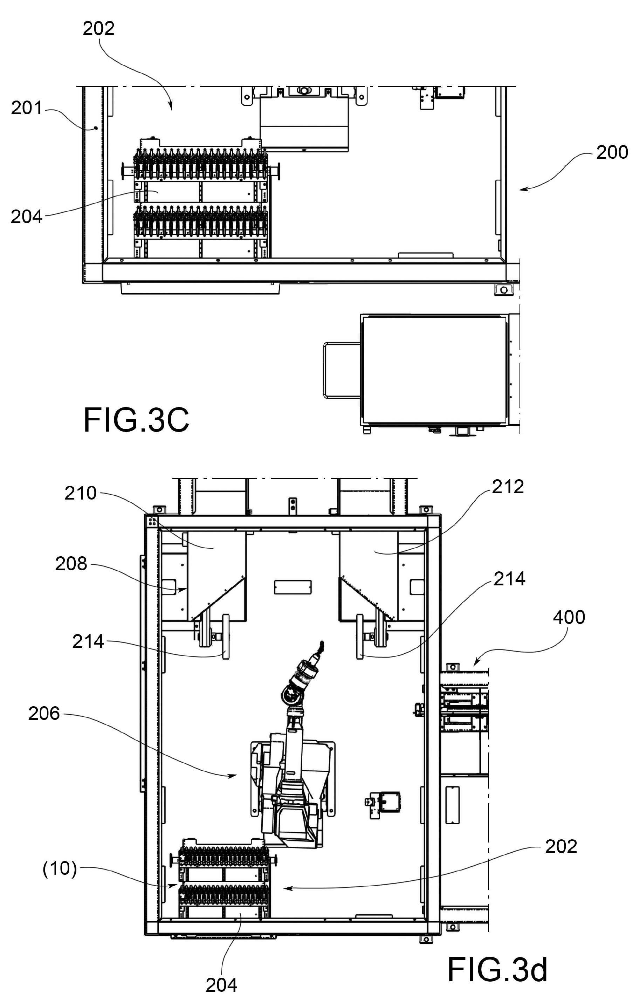

In a subsequent loading step, the loading carriage 204 is loaded with a plurality of semi-processed rods 10 (Figure 3b), preferably manually by an operator. In a subsequent entering step, the loading carriage 204 is actuated and enters into the grinding station 201 (Figure 3c).

The grinding station 200 further comprises a first robot 206 and a grinding unit 208, for example comprising two grinding stations 210, 212 placed side by side, provided with grinding wheels 214.

In a picking step, the first robot 206 picks a single semi-processed rod 10 from the carriage 204 and brings it closer to the grinding unit 208 or to a first checking station 600 for detecting that the semi-processed rod 10 is gripped by the first robot 206.

Then in a grinding step, the first robot 206 moves the semi-processed rod 10 next to the grinding unit 208 to perform the expected grinding processes, by means of the wheels 214 (Figure 3d).

At the end of the grinding step, the first robot 206 places the ground rod 10' on a shifter unit 400 of the finishing apparatus 100, adapted to receive a plurality of ground rods 10' and to transport them to the polishing station 300.

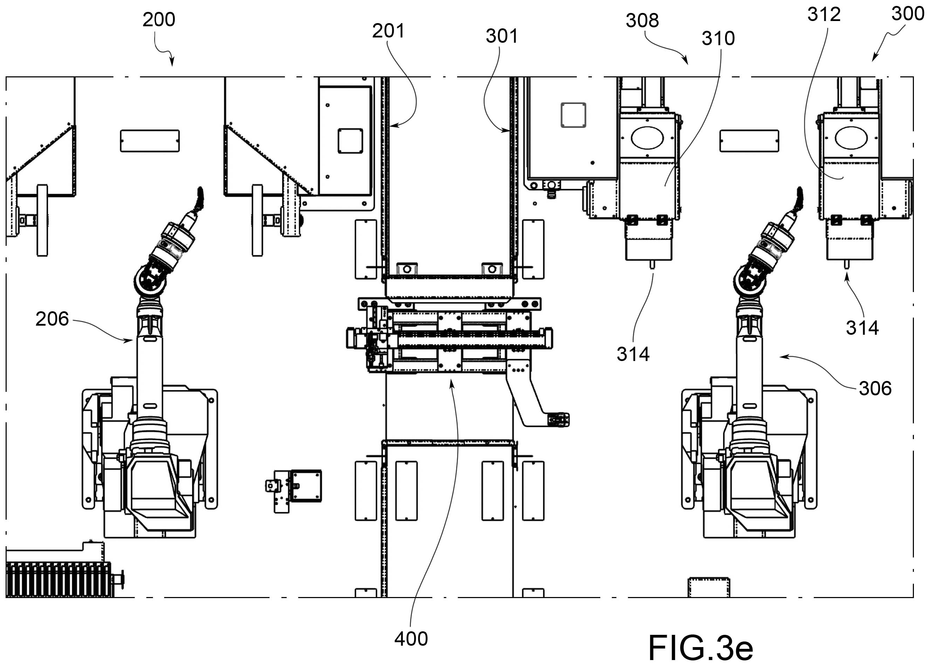

For this purpose, the shifter unit 400 comprises a shifter carriage 402, movable between the grinding station 200 and the polishing station 300, e.g., translationally actuatable, for example pneumatically, along a passage direction W, e.g., orthogonal to the entering direction Y. When the shifter carriage 402 inside the grinding island 200 contains a predefined number of ground rods, it is moved to pass inside the polishing station 300 (Figure 3e).

When the loading carriage 204 inside the grinding booth 201 is empty, it returns outside the grinding booth 201, ready to be loaded again.

The polishing station 300 preferably comprises a polishing booth 301 which delimits it peripherally and preferably at the top.

The polishing station 300 further comprises a second robot 306 and a polishing unit 308, for example comprising two polishing stations 310, 312 placed side by side, provided with polishing wheels 314.

In a picking step, the second robot 306 picks a single ground rod 10' from the shifter carriage 402 and brings it closer to the polishing unit 308 or to a second checking station 700 for checking the correct spatial arrangement of the ground rod 10'.

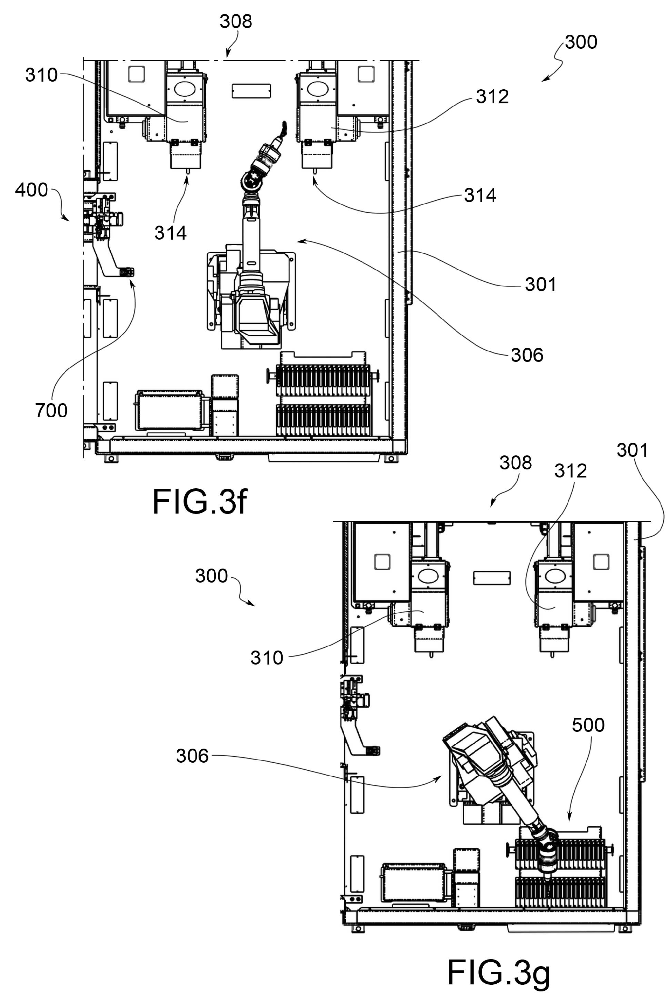

Then, in a polishing step, the second robot 306 moves the ground rod 10' next to the polishing unit 308 to perform the expected polishing processes, by means of the polishing wheels 314 (Figure 3f).

Preferably, once the polishing step has ended, in a tolerance checking step, the second robot 306 then brings the clean rod 10" to a third detection station 900.

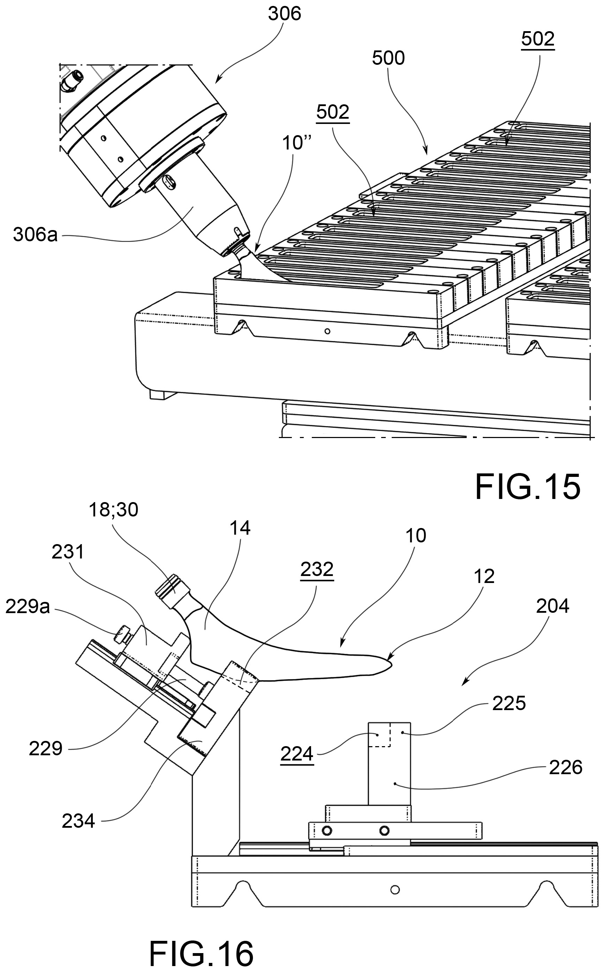

The second robot 306 then places the clean rod 10" onto a finished piece store 500, adapted to receive a plurality of clean rods 10" and to transport them out of the polishing station 300 (Figure 3g and Figure 15).

For this purpose, the finished piece store 500 comprises a plurality of store seats 502, where each store seat 502 is suitable for individually accommodating a clean rod 10''; generally, the store seats 502 are covered by a soft cloth to cushion the support of the clean rod in the respective store seat.

When the shifter carriage 402 inside the polishing booth 301 is empty, it returns to the grinding station 200, ready to be loaded again with additional ground rods 10'.

The grinding station and the polishing station each comprise their own dust suction system. Moreover, since some semi-processed rods are made of titanium alloy, the grinding station 200 comprises a fire prevention system. Advantageously, the two stations are separated from each other by the respective booths, so that the grinding processes on titanium alloy rods do not represent a fire risk for the polishing processes, during which a highly flammable fine dust is generated.

Advantageously, for this purpose, the two stations cooperate with each other by means of the shifter unit accessible for the first robot of the grinding station through a first window obtained in the booth of the grinding station and accessible for the second robot through a second window obtained in the booth of the polishing station.

### Loading unit

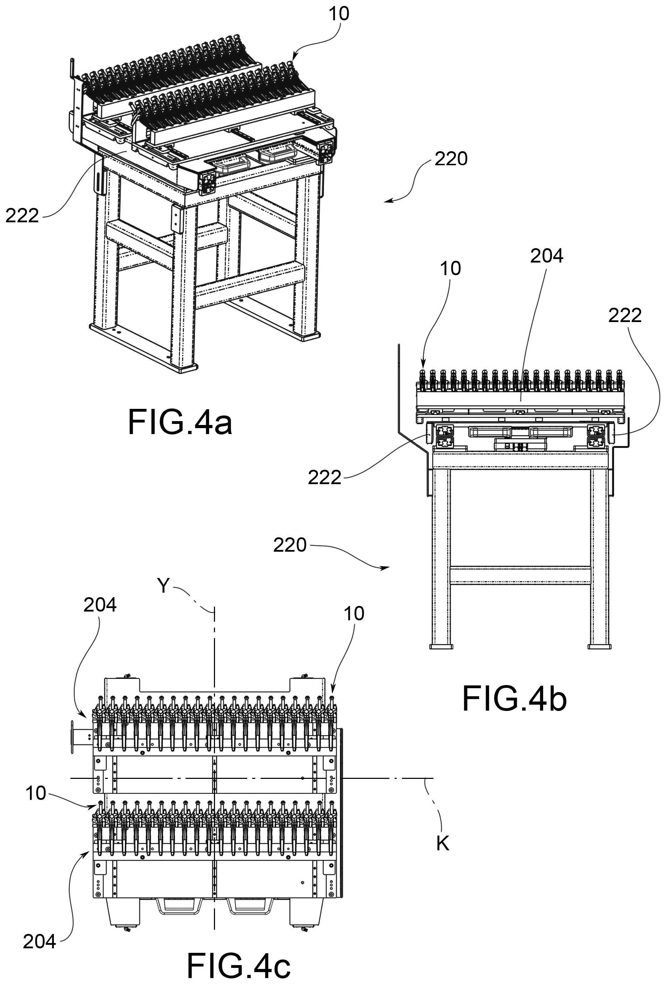

The loading unit 202 comprises a frame 220, rails 222 supported by the frame 220, which cross an inlet window of the booth 201 of the grinding station 200, at a predefined height, and at least one loading carriage 204 slidingly supported by the rails 222 and translationally actuatable along an entering direction Y (Figures 4a, 4b, 4c) between an external position, outside the grinding station, and an internal position, inside the grinding station.

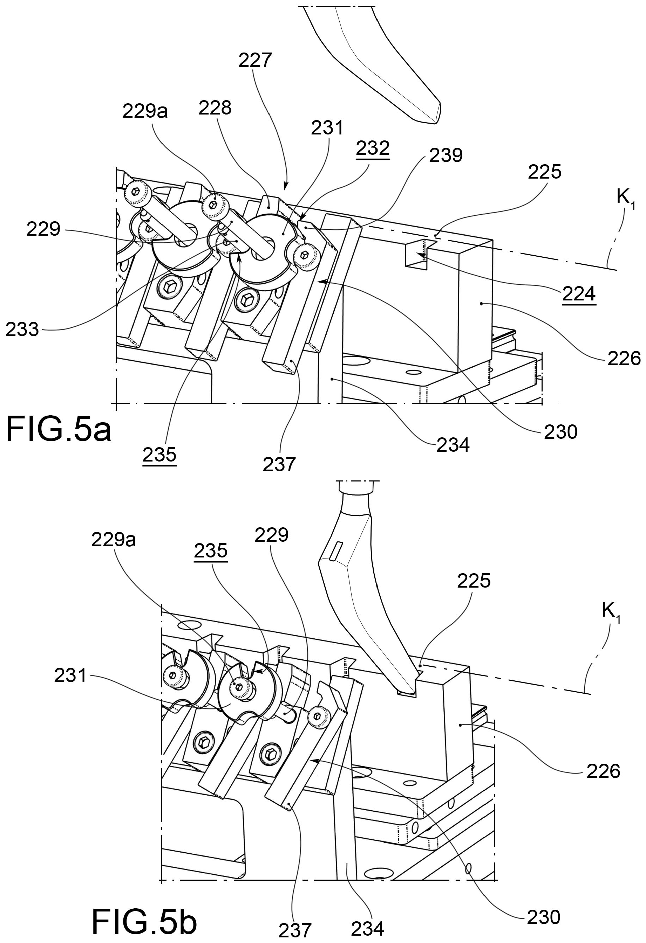

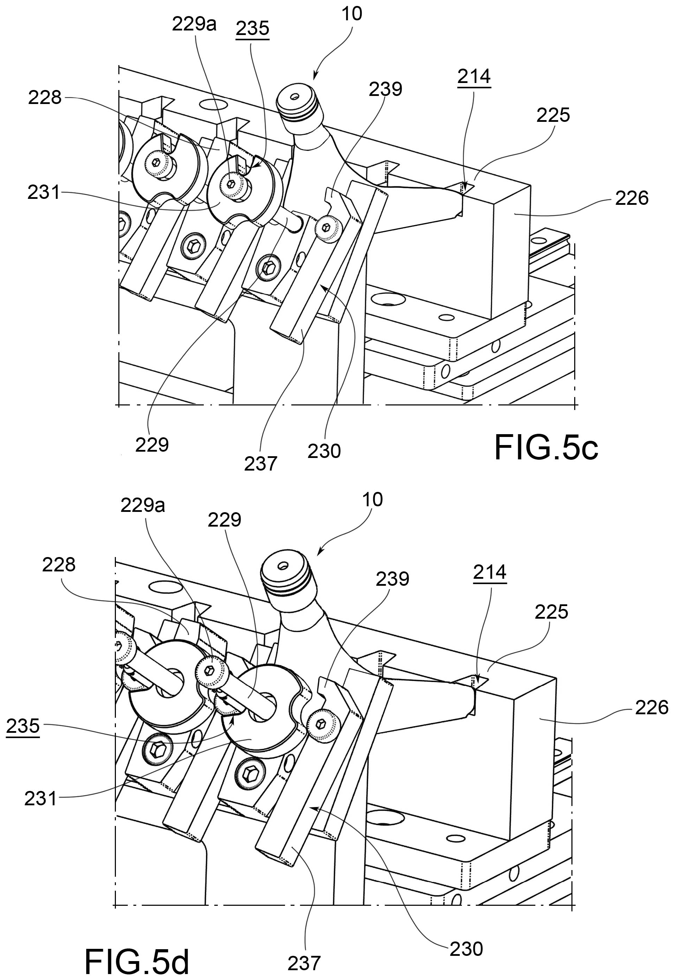

The loading carriage 204 further comprises first positioning means adapted to individually lock a semi-processed rod 10 in a predefined position, in a releasable manner to be gripped by the first robot 206 (Figures 5a, 5b, 5c). Once arranged on the loading carriage 204, inside the grinding station 200, the semi-processed rods are side by side facing one another along a first transverse direction K, e.g., orthogonal to the entering direction Y, parallel to one another and to a first axial direction Q orthogonal to the transverse direction K.

Said first positioning means comprise a first axial positioning device comprising a plurality of tip seats 224, where each tip seat 224 is suitable for housing the lower end 12 of an individual semi-processed rod 10, provided with a bottom wall 225 to simultaneously form an abutment of said lower end 12 in the first axial direction Q. The tip seats 224 are side by side and transversely spaced apart, i.e., along the first transverse direction K, preferably obtained in a first crosspiece 226 of the loading carriage 204.

The first axial positioning device further comprises an axial pusher 227 adapted to operate against the shoulder 17 of the semi-processed rod 10 to push the semi-processed rod 10 in the first axial direction Q. For this purpose, the axial pusher 227 comprises a sliding pin 229 and a base 231 sliding on the pin 229 and rotatable thereon, a locking pin 233 placed alongside the sliding pin 229 and adapted to be accommodated in a locking seat 235 of the base 231, and first elastic axial thrust means adapted to operate permanently on the base 231 to push it towards the semi-processed rod 10.

For example, said first axial thrust means comprise a permanently compressed spring between a head 229a of the sliding pin 229 and the base 231.

Said first positioning means finally comprise a first transverse positioning device comprising a plurality of fixed transverse abutments 228, a plurality of movable transverse pushers 230 and first elastic transverse-thrust means, operating on the transverse pushers 230 along the first transverse direction K; each transverse abutment 228 cooperates with a respective transverse pusher 230 and a head seat 232 is formed between a transverse abutment 228 and the respective transverse pusher 230 for accommodating the upper portion 14 of the semi-processed rod 10.

The transverse pusher 230, for example consisting of a hinged lever 237 provided with a finger 239 projecting towards the transverse abutment 228, on which the elastic transverse thrust means operate, pushes the upper portion 14 of the semi-processed rod 10 to abut against the related transverse abutment 228.

The head seats 232 are side by side and spaced apart transversely along the first transverse direction K, axially spaced apart from the respective tip seats 224, preferably obtained in a second crosspiece 234 of the loading carriage 204.

In an opening configuration of the axial positioning device (Figure 5b), the base 229 is in a first angular position in which the locking pin 233 is outside the locking seat 235 of the base 231 and is positioned along the sliding pin 229 in a distal position from the head seat 232, to easily allow for the insertion of the semi-processed rod 10. In said distal position, the base 231 abuts against the locking pin 233, pushed by the bias of the first axial thrust means.

By rotating the base 231 from the first angular position, the axial positioning device is brought into a closing configuration (Figure 5d), in which the base 229 is in a second angular position in which the locking pin 233 crosses the locking seat 235 of the base 231 and is positioned along the sliding pin 229 in a proximal position with respect to the head seat 232, in order to operate pushing on the semi-processed rod 10 by the bias of said first axial thrust means.

### Step of manually loading the loading carriage

In the loading step, the loading carriage 204 is outside the grinding station, is initially empty and all the axial positioning devices are in the opening configuration, to facilitate the loading of the semi-processed rods 10.

In particular, the operator grips the neck 16 of the upper portion 14 of the semi-processed rod 10, inserts the lower end 12 into the respective tip seat 224 (Figure 5b) and, after slightly rotating the semi-processed rod downwards, inserts the upper portion 14 into the respective head seat 232. When the upper portion 14 is accommodated in the respective head seat 232, the respective transverse pusher 230 pushes the upper portion 14 to abut against the respective transverse abutment 228 by the bias of the first transverse thrust means (Figure 5c).

The operator then acts on the respective axial pusher 227, bringing it from the opening configuration to the closing configuration (Figure 5d); in other words, the operator rotates the base 231 from the first angular position to the second angular position, so as to bring it along the sliding pin 229 into the proximal position in which it abuts against the shoulder 17 of the respective semi-processed rod 10, pushing the lower end 12 against the bottom wall of the tip seat 224.

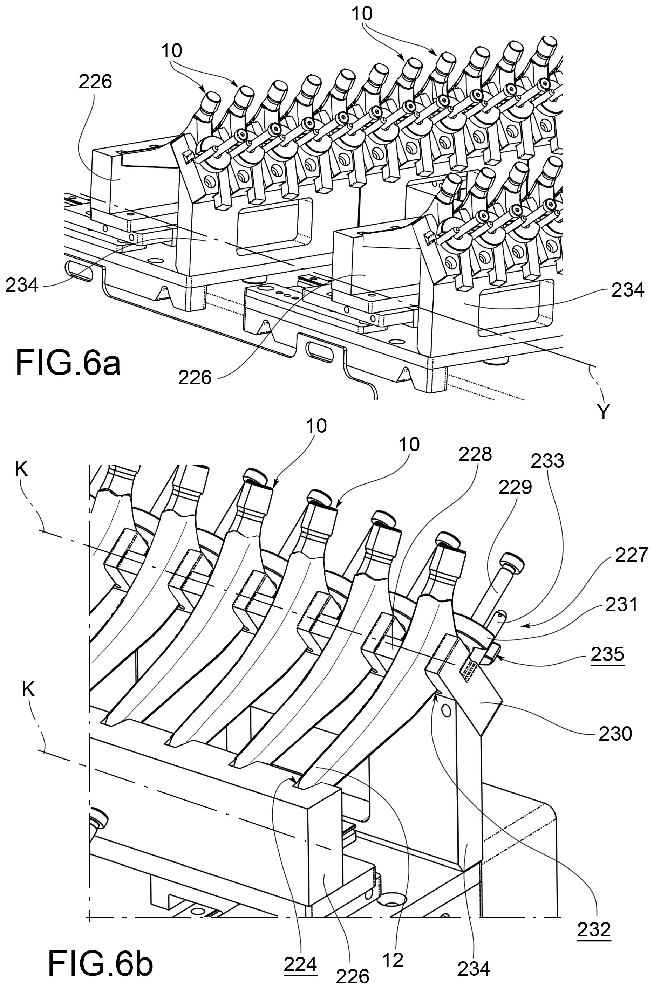

The first transverse positioning device and the first axial positioning device thus allow each semi-processed rod to be positioned exactly in the space, leaving the head 18 of the semi-processed rod 10 free, intended to be gripped by the first robot 206 (Figures 6a and 6b).

According to an embodiment of the invention (Figure 16), the axial positioning device has no locking pin and initially is in an advanced position, proximal to the head seat; during the loading step, the operator moves the base backwards toward a retracted position, overcoming the action of the first axial thrust means, for example using the shoulder of the rod or directly a hand; he/she then inserts the lower end of the semi-processed rod into the tip seat and releases it. The base then pushes the shoulder of the rod, bringing the lower end to abut against the bottom wall of the tip seat.

### Step of picking from the loading carriage

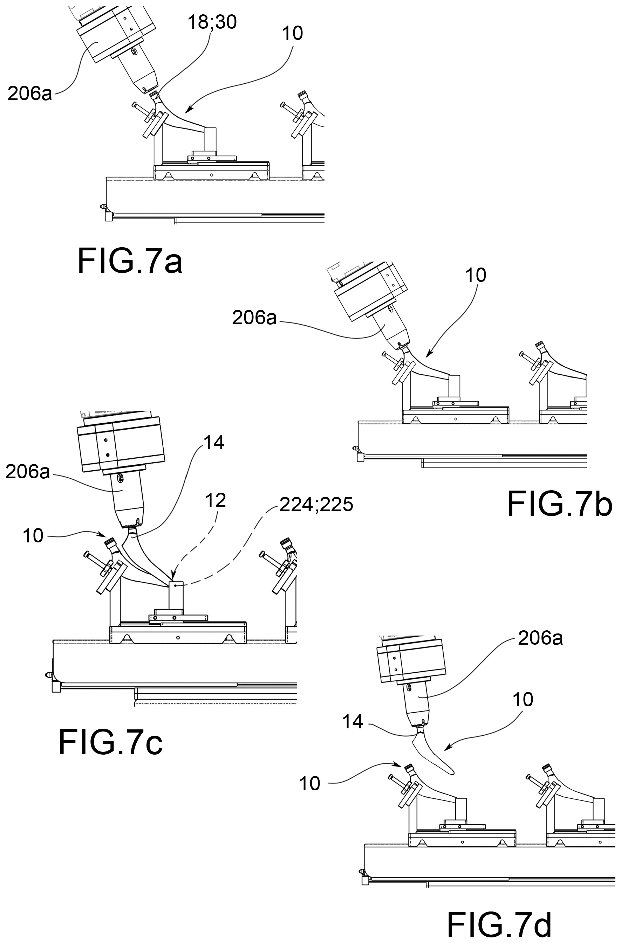

In the step of picking from the loading carriage, the loading carriage 204 is inside the grinding station 200 and contains one or more semi-processed rods in a predefined position; in this step, the first robot 206 picks a single semi-processed rod 10 from the carriage 204 (Figures 7a to 7d).

First, a gripping hand 206a of the first robot 206 moves next to one of said semi-processed rods 10 and grips, for example by means of a radial gripping device, the neck 18 of the semi-processed rod 10 (provided with the cap 30).

The first robot 206 then makes a slight movement so that the semi-processed rod 10 performs a rotation while remaining stuck with the lower end 12 in the tip seat 224, against the bottom wall 225. Therefore, the upper portion 14 of the semi-processed rod 10 is automatically released from the axial pusher 227, since the shoulder 17 slides with respect to the base 231, thus becoming free.

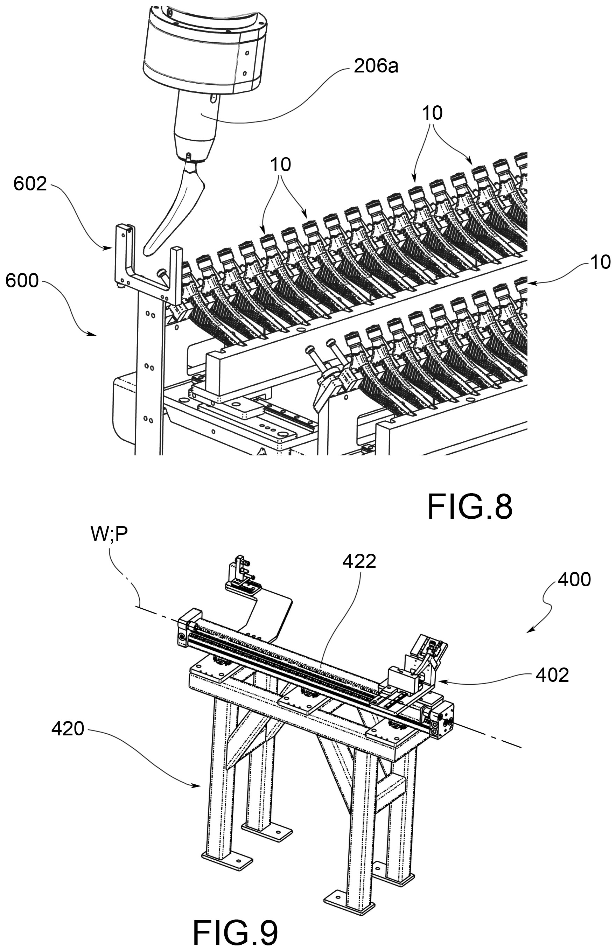

The first robot 206 then brings the semi-processed rod 10 towards the grinding unit 208 or towards a first checking station 600 for checking the presence of the semi-processed rod gripped by the gripping hand 206a (Figure 8).

The first checking station 600 is preferably supported by the frame 220 of the loading unit 200, arranged to the side of the loading carriage 204 and consists of a laser detector 602 which, when the first robot 206 brings the semi-processed rod 10 closer, detects the presence thereof.

### Shifter unit

A shifter unit 400 comprises a frame 420, a rail 422 extending from a first window of the booth 201 of the grinding station 200 to a second window of the booth 301 of the polishing station 300, supported by the frame 420 at a predefined height, and at least one shifter carriage 402 slidingly supported by the rail 422, translationally actuatable along the passage direction W between the grinding station 200 and the polishing station 300 (Figure 9).

When the ground rods are arranged on the shifter carriage, they are side by side facing one another along a second transverse direction P, e.g., parallel to the passage direction W.

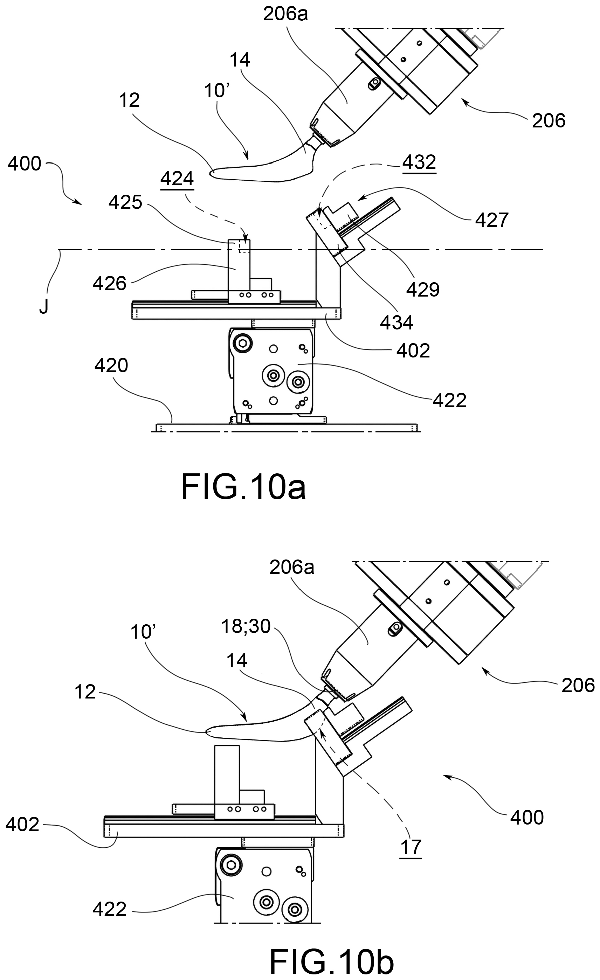

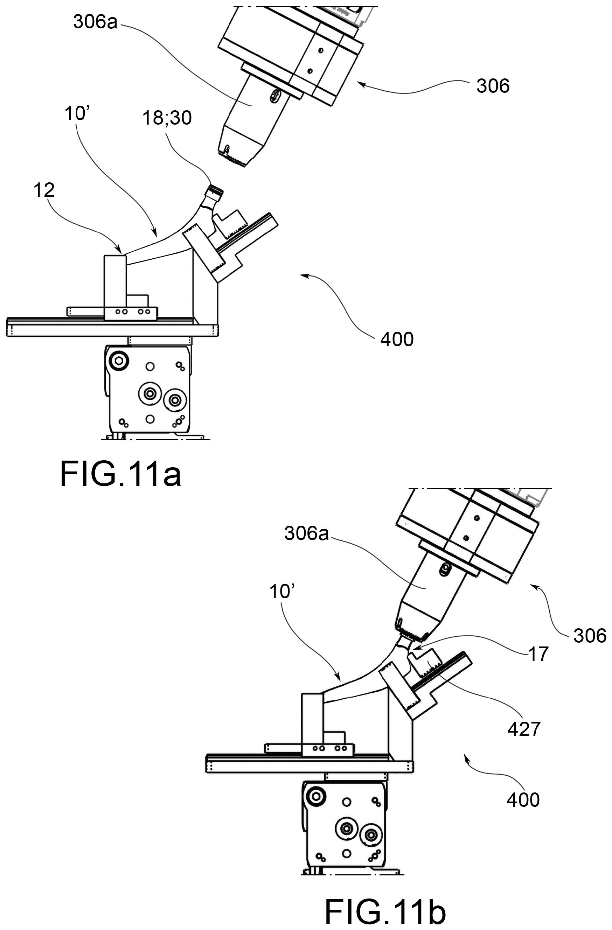

The shifter carriage 402 comprises second automatic positioning means adapted to individually lock a semi-processed rod 10 in a predefined position, actuatable by the first robot 206 during a deposition step and automatically releasable to be gripped by the second robot 306 during a picking step (Figures 10a to 10e and Figures 11a to 11d).

Said second positioning means comprise a second axial positioning device comprising a plurality of tip seats 424, where each tip seat 424 is suitable for housing the lower end 12 of an individual semi-processed rod 10, provided with a bottom wall 425 to simultaneously form an abutment of said lower end 12 in a second axial direction J, which is orthogonal to the second transverse direction P. The tip seats 424 are side by side and transversely spaced apart, preferably obtained in a first crosspiece 426 of the shifter carriage 402.

The second axial positioning device further comprises an axial pusher 427 adapted to permanently operate against the shoulder 17 of the ground rod 10' to push the ground rod 10' in the second axial direction J. For this purpose, the second axial positioning device comprises second elastic thrust means adapted to operate permanently on the axial pusher 427 to push it along the second axial direction J. For example, said second elastic axial thrust means comprise a permanently compressed spring operating on the axial pusher 427.

The second axial positioning device further comprises a head seat 432 to accommodate the upper portion 14 of the ground rod 10'. The head seats 432 are side by side and spaced apart transversely, preferably obtained in a second crosspiece 434 of the shifter carriage 402.

The axial pusher 427 normally abuts against the second crosspiece 434, biased by the second axial thrust means.

### Step of deposition on the shifter carriage

During a deposition step (Figures 10a to 10e), the first robot 206 provided with the gripping hand 206a which grips the neck 18 (with cap 30) of the ground rod 10', moves the ground rod 10' next to the shifter carriage 400 and inserts the upper portion 14 into a head seat 432 (Figure 10b).

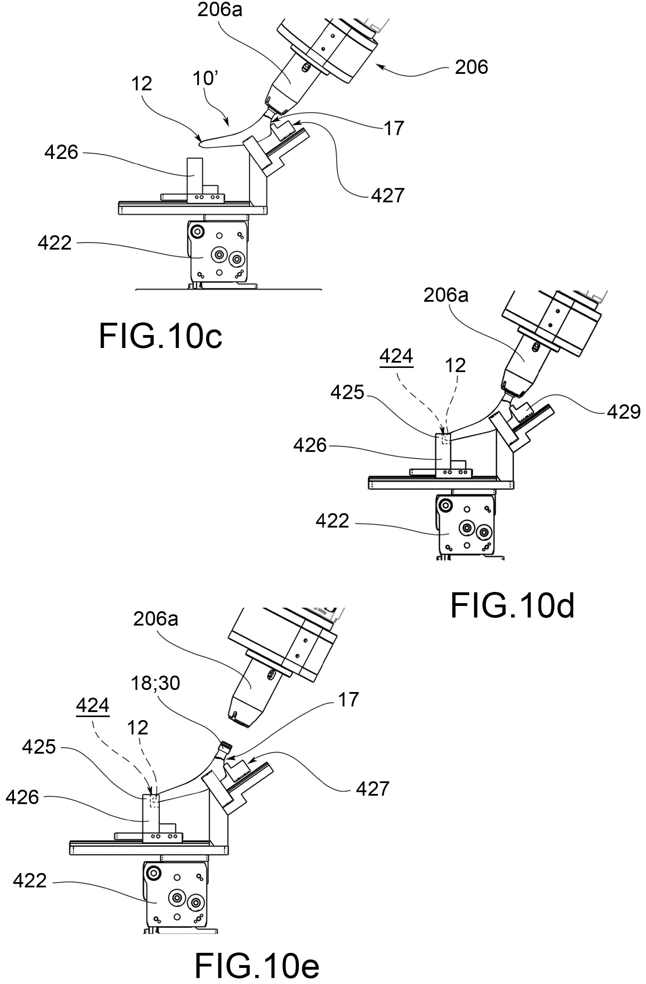

Next, it performs an axial backward movement, by virtue of which the shoulder 17 of the ground rod 10' comes into contact with the axial pusher 427, making it move axially backwards (Figure 10c), i.e., against the bias of the second axial thrust means.

The first robot 206 then performs a movement or rotation by virtue of which the lower end 12 of the ground rod 10' is accommodated in the tip seat 424 (Figure 10d).

The gripping hand 206a of the first robot 206 then releases the grip of the neck 18, so that the axial pusher 427 can axially push the ground rod 10' by operating on the shoulder 17 thereof. The lower end 12 thus abuts against the bottom wall 425 (Figure 10e).

### Step of picking from the shifter carriage

During a picking step (Figures 11a to 11d), the second robot 306, provided with a gripping hand 306a, moves next to the shifter carriage 400 arranged in the polishing station 300 and grips the neck 18 (with cap 30) (Figure 11b).

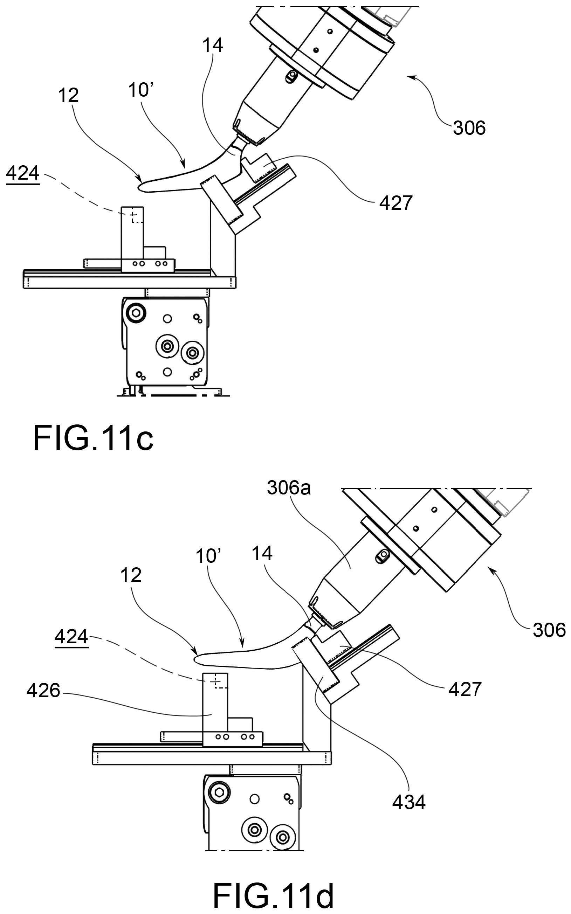

The second robot 306 then performs a movement such as to push the axial pusher 427 backwards, against the bias of the second axial thrust means (Figure 11c), in contact with the shoulder 17 of the ground rod 10'. The lower end 12 thus exits from the tip seat 424. Moreover, the movement is such that the lower end 12 is misaligned from the tip seat 424.

The second robot 306 then performs a return movement that accompanies the axial pusher 427 to the locking position in which it abuts against the second crosspiece 434 of the shifter carriage 400 (Figure 11d). Since the lower end 12 is misaligned from the tip seat 424, the lower end 12 does not fit again into the tip seat 424.

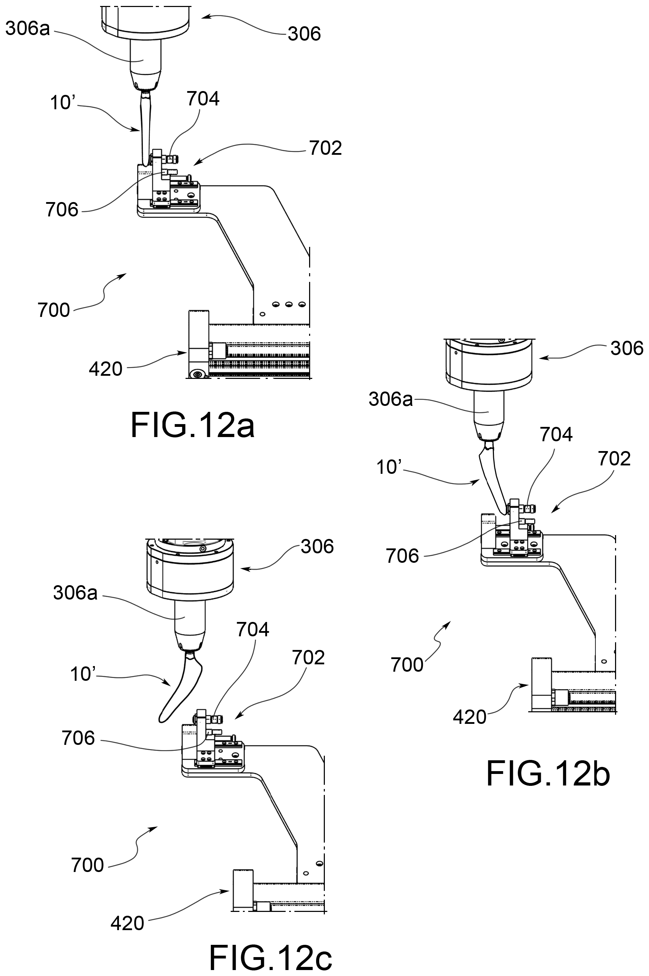

The second robot 306 then brings the ground rod 10' towards the polishing unit 308 or towards a second checking station 700 for checking the spatial arrangement of the ground rod 10' brought by the second robot (Figures 12a to 12c).

The second checking station 700 is preferably supported by the frame 420 of the shifter unit 400, placed in the polishing station 300, and consists of a detector 702 comprising for example two pressure micro-sensors 704, 706 placed on respective slides which are movable independently of each other.

Once the ground rod 10' has been moved next to the detector 702, if the spatial arrangement of the ground rod 10' is correct, i.e., it is aligned with the axis of the gripping hand 306a, the two micro-sensors 704, 706 are activated but not displaced (Figure 12a); if the spatial arrangement is not correct, i.e., the ground rod 10' is misaligned from the axis of the gripping hand 306a, the two micro-sensors 704, 706 are activated and displaced (Figure 12b) or are not activated and not displaced (Figure 12c).

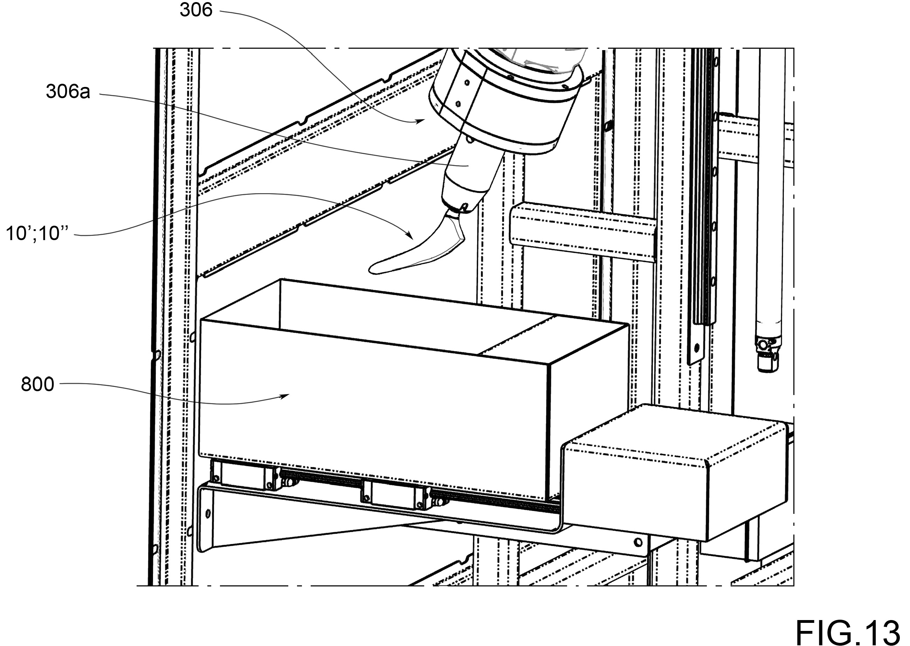

If the spatial arrangement is not correct, the ground rod 10' is discarded, i.e., taken by the second robot 306 to a waste store 800 (Figure 13). If the spatial arrangement is correct, the second robot 306 takes the ground rod 10' towards the polishing unit 308.

### Tolerance checking step

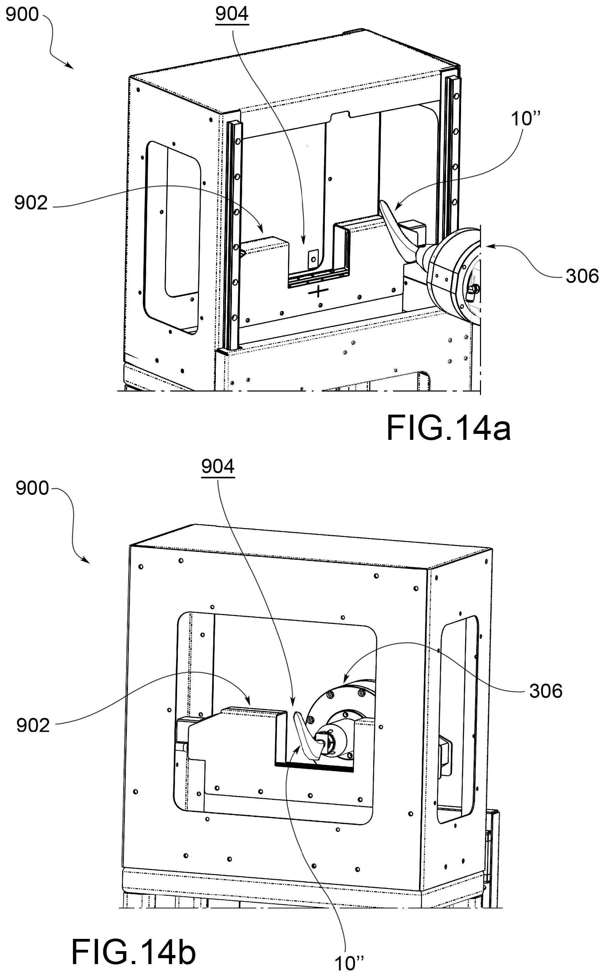

Once the polishing processes have been carried out, the second robot 306 brings the clean rod 10" towards a third checking station 900 (Figure 14a) to check the dimensional and geometric tolerances of the neck 18 of the clean rod 10".

The third checking station 900, arranged in the polishing station 300, comprises a laser detector 902, provided with a detection region 904 accessible for the clean rod 10" and crossed by a laser beam.

The second robot 306 positions the clean rod 10" in the detection region 904, so that the laser beam is incident on the lateral surface of the neck 18 of the clean rod 10" (Figure 14b) and makes the clean rod 10" carry out a rotation about the central rotation axis of the gripping hand, so that the laser beam traces a complete circumferential line on the lateral surface of the neck 18. Based on the detections thus acquired, it is determined whether the external lateral surface of the neck 18 meets the desired dimensional and geometric tolerances.

If the requirements are not met, the clean rod 10" is discarded, i.e., taken by the second robot 306 to the waste store 800 (Figure 13). If the requirements are met, the second robot 306 takes the clean rod 10" to the finished piece store 500 (Figure 15).

The finished piece store 500 is transportable outside the polishing station 300 or is accessible from the outside for picking the clean rods.

Innovatively, the present invention meets the needs of the industry and overcomes the drawbacks mentioned with reference to the prior art, since it allows greatly reducing the times for grinding and polishing prosthesis components, while ensuring high degrees of surface finish and dimensional and geometric tolerances.

Advantageously, the apparatus according to the present invention further allows carrying out the grinding and polishing processes, generally performed by suppliers, directly at the manufacturing company with apparent logistic advantages such as certain production times, established and over-time constant quality level, traceability of batches and waste, resulting in an improvement of the technological know-how.

From this perspective, the loading unit and the shifter unit advantageously minimize the dimensions of the apparatus and are also easy to use by non-specialized operators.

In particular, advantageously, some sensitive parts of the components are subject to particular quality controls, such as for checking the dimensional and geometric tolerances of the rod head.

For this purpose, advantageously, the loading unit and the shifter unit allow arranging the components to be processed directly in the space, so that the gripping and release by the robots is facilitated; this contributes to better carrying out the grinding and polishing processes, so as to comply with the very strict tolerances prescribed.

According to a further advantageous aspect, the processes take place under safe conditions, despite the processing of titanium or titanium alloy components, and in a healthy environment due to the suction of dust.

## Claims

1. A finishing apparatus (100) for carrying out finishing surface operations on components (10, 10', 10") of a biomedical prosthesis, comprising:
- a grinding station (200) for carrying out grinding operations, comprising a booth (201), a first window through the booth (201,) and a dust suction system;
- a polishing station (300) for carrying out polishing operations, comprising a booth (301), a second window through the booth (301), and a dust suction system;
- a shifter unit (400) operating between the first window and the second window to transport the components from the grinding station to the polishing station.

2. A finishing apparatus according to claim 1, wherein the grinding station (200) comprises a fire prevention system.

3. A finishing apparatus according to claims 1 or 2, comprising a loading unit (202) for introducing a plurality of semi-processed rods (10) into the grinding station (200), wherein the loading unit (202) comprises a loading carriage (204) being movable, e.g., translatable, for example by means of pneumatic actuation.

4. A finishing apparatus according to anyone of the preceding claims, wherein the grinding station (200) comprises a first robot (206) and a grinding unit (208), for example comprising two grinding stations (210,212) placed side by side, provided with grinding wheels (214).

5. A finishing apparatus according to claim 4, wherein the grinding station (200) comprises a first checking station (600) for detecting that the semi-processed rod (10) is gripped by the first robot (206).

6. A finishing apparatus according to anyone of the preceding claims, wherein the shifter unit (400) comprises a shifter carriage (402), movable between the grinding station (200) and the polishing station (300), e.g., translationally actuatable, for example pneumatically.

7. A finishing apparatus according to anyone of the preceding claims, wherein the polishing station (300) comprises a second robot (306) and a polishing unit (308), for example comprising two polishing stations (310,312) placed side by side, provided with polishing wheels (314).

8. A finishing apparatus according to claim 7, wherein the polishing station (300) comprises a second checking station (700) for checking the spatial arrangement of the ground rod (10') brought by the second robot.

9. A finishing apparatus according to claim 7 or 8, wherein the polishing station (300) comprises a third checking station (900) for checking the dimensional and geometric tolerances of the clean rod (10"), for example a laser detector (902).

10. A finishing apparatus according to anyone of the preceding claims, comprising a finished piece store (500) for receiving a plurality of clean rods (10") and for transporting them out of the polishing station (300), wherein the finished piece store (500) comprises a plurality of store seats (502), wherein each store seat (502) is suitable for individually accommodating a clean rod (10").
